# FASCICULE DE BREVET EUROPEEN

(11) **EP 2 608 770 B1**
(45) Date de publication et mention de la délivrance du brevet: **25.01.2017**
(21) Numéro de dépôt: 11757374.1
(22) Date de dépôt: 01.08.2011
(51) Int. Cl.: A61K 9/00, A61K 33/00, A61K 9/72, A61P 25/00

(54) **MEDICAMENT GAZEUX INHALABLE A BASE DE KRYPTON POUR LE TRAITEMENT DES NEURO-INTOXICATIONS**
INHALIERBARER GASFÖRMIGER WIRKSTOFF MIT KRYPTON ZUR BEHANDLUNG VON NERVENVERGIFTUNG
INHALABLE GASEOUS DRUG CONTAINING KRYPTON FOR TREATING NEURO-INTOXICATION

(30) Priorité: 24.08.2010 FR 1056715
(43) Date de publication de la demande: 03.07.2013
(73) Titulaire: L'Air Liquide Société Anonyme pour l'Etude et l'Exploitation des Procédés Georges Claude, 75007 Paris (FR)
(72) Inventeur: LEMAIRE, Marc, F-75014 Paris (FR)
(74) Mandataire: Pittis, Olivier
(86) Numéro de dépôt international: PCT/FR2011/051849
(87) Numéro de publication internationale: WO 2012/025677

(56) Documents cités:
- EP-A1- 1 994 935
- EP-A1- 2 014 293
- WO-A2-2008/122654
- JAWAD NOORULHUDA ET AL: "Neuroprotection (and lack of neuroprotection) afforded by a series of noble gases in an in vitro model of neuronal injury", NEUROSCIENCE LETTERS, vol. 460, no. 3, septembre 2009 (2009-09), pages 232-236, XP002607569, ISSN: 0304-3940
- CULLEN S C ET AL: "THE ANESTHETIC PROPERTIES OF XENON IN ANIMALS AND HUMAN BEINGS, WITH ADDITIONAL OBSERVATIONS ON KRYPTON", SCIENCE, AMERICAN ASSOCIATION FOR THE ADVANCEMENT OF SCIENCE, WASHINGTON, DC; US, vol. 113, 18 mai 1951 (1951-05-18), pages 580-582, XP008035193, ISSN: 0036-8075, DOI: DOI:10.1126/SCIENCE.113.2942.580

## Description

L'invention porte sur l'utilisation de krypton gazeux en tant que médicament inhalable destiné à traiter ou à prévenir une pathologie ou un trouble à effet neurotoxique, c'est-à-dire une neuro-intoxication.

Une neuro-intoxication peut se définir par une augmentation de l'un des neurotransmetteurs cérébraux tels que : dopamine, sérotonine, taurine, acide γ-amino-butyrique (GABA), noradrénaline, glutamate...

La neuro-intoxication est une situation critique dont la cause peut être aussi bien une ischémie, un syndrome d'ischémie-reperfusion, un traumatisme, une ingestion aiguë de drogues (amphétamines...), une addiction...

De façon particulièrement intéressante, il a été montré que l'inhibition spécifique des transporteurs du glutamate permet de diminuer à la fois l'hyperactivité (David et al. Neuropharmacology, 2001, vol. p. 409-411) et l'augmentation de glutamate, mais pas de dopamine (Del Arco et al., Neuropharmacology, 1999, vol. 38, p. 943-954).

Par ailleurs, des travaux récents, réalisés in vitro, ont montré que le xénon et le protoxyde d'azote (N₂O) peuvent se comporter comme des antagonistes des récepteurs glutamatergiques au N-Méthyl-D-Aspartate (NMDA) ; Franks et al., Nature, 1998, vol. 396, p. 324 ; Jevtovic-Todorovic et al., Nature Med., 1998, vol. 4, p. 460- 463.

En outre, dans le cadre de l'étude du système opioïde hyperalgésique endogène dans la réponse placebo négative, F.J. Lichtigfeld et M.A. Gillman, Intern. J. Neuroscience, 1989, vol. 49, p. 71-74 concluent à un effet du protoxyde d'azote sur le sevrage alcoolique un peu meilleur que l'effet placebo, bien que, pour plus de 50% des individus, un effet positif identique a aussi été constaté avec le placebo.

Toutefois, les mêmes auteurs ajoutent, dans Nitrous Oxide and the Aws, p. 785, que l'effet bénéfique du protoxyde d'azote dépendant étroitement de sa concentration car des concentrations anesthésiques ou pré-anesthésiques sont inefficaces, voire même contre-productives dans certains cas ; une concentration analgésique étant recommandée.

Dans Postgrad. Med. J, Clinical Toxicology, 1990, vol. 66, p. 543-546, les mêmes auteurs expliquent que les concentrations en protoxyde d'azote peuvent varier de moins de 15% à plus de 70% selon les individus et ce, en fonction de leur degré de dépendance à l'alcool.

Par ailleurs, le document EP-A-1158992 enseigne l'utilisation de xénon ou d'un mélange de xénon avec de l'oxygène, de l'azote ou de l'air pour traiter les neuro-intoxications.

Le document EP-A-1158992 divulgue l'utilisation du xénon en mélange avec de l'oxygène pour le traitement de dommages ischémiques cérébraux et indique que la composition peut comprendre du krypton.

Toutefois, l'utilisation de xénon ou des mélanges décrits par ce document n'est pas totalement satisfaisante en pratique, notamment du fait de l'apparition d'une toxicité pour certaines teneurs en xénon et compte tenu du coût élevé de ce composé ; David et al.; J. Cereb. Blood Flow. Metab., 2003, vol. 23, p. 1168-1173.

Par ailleurs, le document EP-A-1541156 et EP-A-2014293 enseignent l'utilisation d'argon en tant que principe actif, mélangé ou non avec un autre gaz, tel de l'oxygène, de l'azote, du xénon, du krypton ou de l'air, permettant de traiter les neuro-intoxications.

La présente invention s'inscrit dans ce contexte et vise à améliorer les médicaments inhalables existant destinés à prévenir ou traiter efficacement une neuro-intoxication chez l'homme, laquelle se caractérise par un dysfonctionnement cérébral d'un ou de plusieurs systèmes de neurotransmission.

La solution de l'invention porte alors sur une composition gazeuse constituée de krypton en une proportion volumique de 15 à 80 % et d'au moins un composé gazeux additionnel choisi parmi l'oxygène, l'azote et leur mélanges, pour une utilisation en tant que médicament inhalable destiné à prévenir ou à traiter une neuro-intoxication chez l'homme, dans laquelle le krypton gazeux est le principe actif.

Par neuro-intoxication, on entend un trouble, un désordre ou une pathologie du système nerveux central dont l'étiopathogénie implique, au moins pour partie, un processus excito-toxique, notamment un dysfonctionnement de la neurotransmission excitatrice au glutamate ; voir notamment le document Parsons et al., Drug News Perspect., 1998, vol.11, pages 523-569.

En conséquence, entre dans le cadre de la présente invention, le traitement notamment:
- des accidents cérébraux aigus secondaires à des traumatismes crâniens ou à des accidents vasculaires cérébraux (AVC), y compris l'ischémie cérébrale ;
- des effets neurotoxiques de drogues ou autres substances pouvant générer une addiction comme les amphétamines et dérivés amphétaminiques, les substances opiacées et leurs dérivés, la cocaïne et ses dérivés, le tabac, le cannabis ou l'alcool ;
- des maladies neuro-dégénératives comme la maladie d'Alzheimer, la maladie de Parkinson, la maladie (chorée) de Huntington, la sclérose latérale amyotrophique, l'encéphalomyélite aiguë disséminée, la dyskinésie tardive, et les dégénérescences olivopontocérébelleuses ; et
- diverses pathologies psychiatriques ou neurologiques comme les troubles anxieux, les troubles psychotiques, notamment la schizophrénie et l'épilepsie sous ses diverses formes.

Selon le cas, composition gazeuse médicamenteuse de l'invention peut comprendre l'une ou plusieurs des caractéristiques techniques suivantes:
- la neuro-intoxication résulte d'un dysfonctionnement cérébral, à savoir un excès ou une diminution, d'un ou plusieurs systèmes de neurotransmetteurs.
- le mélange contient du krypton en une proportion efficace et suffisante pour agir sur au moins un récepteur cérébral afin de réguler le fonctionnement des systèmes de neurotransmission à la dopamine, au glutamate, à la sérotonine, à la taurine, à l'acétylcholine, au GABA et/ou à la noradrénaline.
- la proportion volumique de krypton dans ledit mélange gazeux est comprise entre 15 et 80 %.
- la proportion volumique de krypton est d'au moins 30% et/ou d'au plus 75% en volume.
- la proportion volumique de krypton est d'au moins 40% en volume.
- le krypton est en mélange gazeux avec au moins un composé gazeux additionnel choisi parmi le xénon, l'argon et le protoxyde d'azote (N₂O), l'oxygène, l'azote ou leurs mélanges, en particulier de l'air.
- le mélange gazeux est un mélange binaire constitué de krypton et d'oxygène pour le reste, de préférence le médicament gazeux est prêt à l'emploi, c'est-à-dire qu'il peut être administré au patient directement sans subir de pré-dilution.
- la neuro-intoxication est choisie parmi les troubles, désordres ou pathologies du système nerveux central dont l'étiopathogènie implique, au moins pour partie, un processus excititoxique, comme les effets neurotoxiques de drogues ou substances pouvant générer un état d'addiction, les accidents cérébraux aigus, les maladies neuro-dégénératives, et diverses pathologies psychiatriques ou neurologiques.

Par "neuro-intoxication engendrant un état d'addiction", on entend un trouble, un désordre ou une pathologie lié aux effets neurotoxiques d'une drogue, molécule ou substance générant une addiction ou une accoutumance chez l'homme ou l'animal. La substance, drogue ou molécule générant l'addiction est choisie dans le groupe formé par les amphétamines et leurs dérivés, les substances opiacées et leurs dérivés, la cocaïne et ses dérivés, le tabac, l'alcool et le cannabis, ou toute autre drogue similaire ou analogue. Par "accident cérébral aigu", on entend un trouble, un désordre ou une pathologie consécutif à un évènement brutal et soudain d'origine exogène ou endogène. L'évènement exogène peut être un trauma crânien, alors que l'évènement endogène peut être la rupture ou l'occlusion d'une artère ou d'un vaisseau sanguin cérébral. Par maladie neuro-dégénératives, on entend un trouble, un désordre ou une pathologie lié à la dégénérescence et la mort de certains neurones cérébraux.
- le médicament inhalable est conditionné à une pression de 2 bars à 350 bars, de préférence entre 2 bars et 200 bars.
- elle est formée uniquement de krypton et d'oxygène.
- il est formé de 20 à 80 % en volume de krypton et d'oxygène pour le reste, de préférence au moins 30 et/ou au plus 75% de krypton (% en volume). Dans tous les cas, les proportions de krypton et/ou d'oxygène dans le mélange gazeux pourront être ajustées en fonction de la durée du traitement.

D'une façon générale, lors du traitement, l'administration de krypton chez l'homme peut se faire au moyen d'un ventilateur, d'un nébuliseur ou spontanément avec des bouteilles pré conditionnées, raccordés à un masque facial ou nasal, ou des lunettes nasales.

La durée d'administration sera choisie au cas par cas en fonction de l'importance de la maladie ou du trouble affectant le patient considéré, par exemple le krypton pourra être administré pendant une durée de quelques minutes à quelques dizaines de minutes, voire d'heures, par exemple moins d'une heure, et à une fréquence pouvant atteindre une à plusieurs fois par jour ou par semaine, par exemple 1 fois par jour pendant 2 semaines.

Le krypton ou mélange gazeux à base de krypton est préférentiellement conditionné en bouteille de gaz sous pression ou sous forme liquide, par exemple dans une bouteille d'un à plusieurs litres (contenant en eau) et à une pression comprise entre 2 et 300 bar.

Le krypton ou mélange gazeux à base de krypton peut se présenter sous forme « prêt à l'emploi », par exemple en pré-mélange avec de l'oxygène, ou alors être mélangé sur site lors de son utilisation, notamment avec de l'oxygène et éventuellement un autre composé gazeux.

Autrement dit, l'idée à la base de la présente invention est donc que les propriétés du krypton peuvent être utilisées, pour limiter les effets excitateurs de neurotransmetteurs cérébraux, tel le du glutamate, afin de prévenir et/ou de traiter les neuro-intoxications, notamment les effets neurotoxiques de drogues ou substances générant une addiction, telles que les amphétamines et leurs dérivés, les substances opiacées et leurs dérivés, la cocaïne et ses dérivés, le tabac, l'alcool, le cannabis ou tout autre substance engendrant une dépendance.

De façon générale, le médicament gazeux selon l'invention est administrable au patient par ses voies aériennes supérieures, c'est-à-dire par inhalation via son nez et/ou sa bouche, au moyen d'un dispositif d'administration adapté comprenant une interface respiratoire patient, tel qu'un masque respiratoire ou une sonde trachéale, une ou plusieurs canalisations d'alimentation servant à acheminer le médicament gazeux depuis une source contenant ledit médicament jusqu'à l'interface, et un ventilateur médical servant à envoyer et/ou extraire le gaz du patient.

La présente invention va être mieux comprise grâce aux exemples illustratifs suivants.

### Exemples

Des essais ont été réalisés pour démontrer que le krypton présente un effet protecteur cérébral de par sa capacité à protéger la cellule du phénomène de la mort cellulaire programmée ou apoptose, qui est la conséquence ultime des neuro-intoxications telles que décrites dans le syndrome d'ischémie-reperfusion, d'addictions...

Les conditions expérimentales mimaient les dégâts cellulaires liées à l'ischémie-reperfusion, via les effets cytotoxiques et antiprolifératifs de la staurosporine qui est un inhibiteur global de la tyrosine kinase interrompant tous les signaux trophiques; de la roténone qui est un inhibiteur du complexe I de la chaîne respiratoire; de l'antimycine A qui est un inhibiteur du complexe III de la chaîne respiratoire; de la ménédione qui est un agent redox inhibant la production d'espèces oxygène-réactives sur la chaine respiratoire) ;et de la mitoxanthrone qui est une anthracycline générant des anomalies du DNA.

Les effets ont été évalués à des concentrations proches à une dose semi létale, puis à 1/10, 1/3, X 3 et X 10 ladite dose.

Pour ce fait, les cellules ont été maintenues dans différents milieux de conservation, type milieu seul, milieu sérum free, milieu sérum et nutriment free (condition reproduisant la déplétion en nutriments et facteurs trophiques), milieu contenant de la rapamycine pour réaliser un contrôle positif de l'induction de l'autophagie ; ou du nocodazole pour réaliser uncontrôle positif de l'arrêt de la mitose cellulaire.

Toutes les expériences ont été menées sous atmosphères gazeuses de type krypton ou autres gaz appliquées à des fins d'analyse statistique.

Les mesures ont été réalisées après 6 et 16 heures d'incubation.

Les cellules suivantes ont été testées et étudiées :
- U2OS en présence de: GFP-histone H2B (protéine verte en fluorescence générée pour marquer la chromatine et permettre de suivre le cycle de la division cellulaire ainsi que la condensation nucléaire du processus d'apoptose); Red-centrin (protéine rouge en fluorescence marquant les centrosomes) afin de mesurer la prolifération et l'apoptose en culture cellulaire.
- U2OS en présence de GFP-HMGB1 (protéine relarguée par le noyau des cellules nécrotiques), et de DAPI (marquage de la chromatine) pour évaluer simultanément la nécrose (relarguage de GFP-HMGB1) et l'apoptose (définit par le niveau de condensation de la chromatine), qui peut être primaire (pas de relarguage de GFP-HMGB1) ou secondaire (relarguage de GFP-HMGB1).
- U2OS en présence de GFP-LC3 (protéine capable de se redistribuer: de diffus à des amas localisés punctiformes dénommés autophagosomes, quand l'autophagie est induite) afin de mesurer l'autophagie.
- U2OS en présence de GFP-G3B (protéine capable de se redistribuer: de diffus à des amas localisés punctiformes dénommés granules de stress, quand le stress endoplasmique (ER) est induit) afin de mesurer le stress ER.
- U2OS en présence de 5-ethyl-2'deoxyuridine (une base thymidine analogue qui s'incorpore dans le DNA de cellules en prolifération) révélée par Click-IT-Alexa-Fluor azide (sonde moléculaire afin de rendre fluorescent le 5-ethyl-2'doxyuridine) et DAPI afin d'étudier le contenu du DNA.

Les cellules ont été ensuite analysées dans un automate BD Pathway Fluorescence Microscope afin de déterminer les paramètres suivants :
- apoptose cellulaire définit par GFP-H2B ou DAPI-condensation chromatine mesurable),
- autophagie définit par GFP-LC3 puncta,
- nécrose définit par l'exsudation de HMGB1 du noyau,
- aberrations mitotiques définit par GFP-H2B et Red-Centrin,
- niveau de stress ER définit par GFP-G3B puncta,
- cycle cellulaire définit par l'incorporation de 5-ethyl-2'doxyuridine dans les cellules en phase S, ainsi que l'analyse du DNA.

De plus, les cellules U20S ont été marquées avec plusieurs fluorochromes déterminant l'état fonctionnel des mitochondries, en particulier l'ester de tetramethyl rhodamin, cation lipophilique mesurant le potentiel transmembranaire interne de la mitochondrie, la génération d'espèces oxygène réactive tel que dehydroethidium, qui oxydé génère le produit fluorescent ethidium, ou le contenu en glutathion tel que thiol-reactive fluorochrome monochlorobiman.

Au final, bien que le mécanisme d'action par lequel le krypton est capable d'opérer une neuroprotection ne soit pas encore établi avec certitude, les résultats de ces essais montrent que les cellules conservées en atmosphère formée de krypton conservent leurs capacités fonctionnelles ainsi que leur intégrité physiques puisque le krypton diminue les signes d'apoptose cellulaire, c'est-à-dire les conséquences lésionnelles lors d'une neuro-intoxication telles que l'ischémie-reperfusion, l'addiction, les maladies neuro-dégénératives...

Dans tous les cas, ces essais montrent que le krypton peut être utilisé en tant que médicament inhalable pour prévenir ou traiter les conséquences d'une neuro-intoxication.

L'ensemble de ces résultats permet d'envisager un intérêt thérapeutique potentiel du krypton en mélange avec un diluent gazeux dans le traitement des neuro-intoxications cérébrales chez l'homme.

Selon l'invention, le médicament gazeux est préférentiellement un mélange gazeux binaire constitué de krypton et d'oxygène (>21% en volume) pour le reste ou un mélange ternaire constitué de krypton, d'azote et d'oxygène (>21% en volume), de préférence le médicament gazeux est prêt à l'emploi.

En particulier, le mélange gazeux est formé d'au moins 20% et/ou d'au plus 80% en volume de krypton, et d'azote et d'oxygène pour le reste, de préférence d'au moins 30% et d'au plus 75% de krypton (% en volume).

## Revendications

1. Composition gazeuse constituée de krypton en une proportion volumique de 15 à 80 % et d'au moins un composé gazeux additionnel choisi parmi l'oxygène, l'azote et leur mélanges, pour une utilisation en tant que médicament inhalable destiné à prévenir ou à traiter une neuro-intoxication chez l'homme, dans laquelle le Krypton gazeux est le principe actif.

2. Composition gazeuse pour utilisation selon la revendication 1, **caractérisée en ce que** la neuro-intoxication résulte d'un dysfonctionnement cérébral d'un ou plusieurs systèmes à neurotransmetteurs.

3. Composition gazeuse pour utilisation selon l'une des revendications 1 ou 2, **caractérisée en ce qu'**elle contient du Krypton en une proportion efficace et suffisante pour agir sur au moins un récepteur cérébral afin de réguler le fonctionnement des systèmes de neurotransmission à la dopamine, glutamate, sérotonine, acétylcholine, taurine, GABA et/ou noradrénaline.

4. Composition gazeuse pour utilisation selon l'une des revendications 1 à 3, **caractérisée en ce que** la proportion volumique de Krypton est d'au moins 30%.

5. Composition gazeuse pour utilisation selon l'une des revendications 1 à 4, **caractérisée en ce que** la proportion volumique de krypton est d'au plus 75%.

6. Composition gazeuse pour utilisation selon l'une des revendications 1 à 5, **caractérisée en ce qu'**elle contient de l'air, de préférence le médicament gazeux est prêt à l'emploi.

7. Composition gazeuse pour utilisation selon l'une des revendications 1 à 6, **caractérisée en ce qu'**elle est constituée de krypton et d'oxygène pour le reste ou de krypton, d'azote et d'oxygène.

8. Composition gazeuse pour utilisation selon l'une des revendications 1 à 7, **caractérisée en ce qu'**elle contient au moins 20% en volume d'oxygène.

9. Composition gazeuse pour utilisation selon l'une des revendications 1 à 8, **caractérisée en ce que** la neuro-intoxication est choisie parmi les excito-toxicités engendrant un état d'addiction, les accidents cérébraux aigus, les maladies neuro-dégénératives, et les pathologies psychiatriques ou neurologiques.

10. Composition gazeuse pour utilisation selon l'une des revendications 1 à 8, **caractérisée en ce que** la neuro-intoxication résulte ou est consécutive à un traumatisme crânien, à un accident vasculaire cérébral (AVC), à une ischémie cérébrale, à la prise d'une substance générant une addiction, à la maladie d'Alzheimer, à la maladie de Parkinson, à la chorée de Huntington, à une sclérose latérale amyotrophique, à une encépholomyélite aiguë disséminée, à une dyskinésie tardive, à une dégénéresence olivopontocérébelleuse, à une schizophrénie ou à une épilepsie.

## Patentansprüche

1. Gaszusammensetzung bestehend aus Krypton in einem Volumenanteil von 15 bis 80 % und mindestens einer zusätzlichen gasförmigen Verbindung, ausgewählt aus Sauerstoff, Stickstoff und deren Mischungen, zu einer Verwendung als inhalierbares Arzneimittel, welches dazu bestimmt ist, eine Neurointoxikation beim Menschen zu verhindern oder zu behandeln, in welcher gasförmiges Krypton der Wirkstoff ist.

2. Gaszusammensetzung zur Verwendung
nach Anspruch 1, **dadurch gekennzeichnet, dass** die
Neurointoxikation aus einer Dysfunktion eines oder mehrerer Neurotransmittersysteme im Gehirn resultiert.

3. Gaszusammensetzung zur Verwendung
nach einem der Ansprüche 1 oder 2, **dadurch gekennzeichnet,**
**dass** sie Krypton in einem Anteil enthält, welcher wirksam und ausreichend ist, um auf mindestens einen Rezeptor im Gehirn zu wirken, um die Funktion der Dopamin-, Glutamat-, Serotonin-, Acetylcholin-, Taurin-, GABA- und/oder Noradrenalin-Neurotransmissionssysteme zu regulieren.

4. Gaszusammensetzung zur Verwendung
nach einem der Ansprüche 1 bis 3, **dadurch gekennzeichnet,**
**dass** der Volumenanteil von Krypton mindestens 30 % beträgt.

5. Gaszusammensetzung zur Verwendung
nach einem der Ansprüche 1 bis 4, **dadurch gekennzeichnet,**
**dass** der Volumenanteil von Krypton höchstens 75 % beträgt.

6. Gaszusammensetzung zur Verwendung
nach einem der Ansprüche 1 bis 5, **dadurch gekennzeichnet,**
**dass** sie Luft enthält, wobei das gasförmige Arzneimittel bevorzugt gebrauchsfertig ist.

7. Gaszusammensetzung zur Verwendung
nach einem der Ansprüche 1 bis 6, **dadurch gekennzeichnet,**
**dass** sie aus Krypton und für den Rest aus Sauerstoff oder aus Krypton, Stickstoff und Sauerstoff besteht.

8. Gaszusammensetzung zur Verwendung
nach einem der Ansprüche 1 bis 7, **dadurch gekennzeichnet,**
**dass** sie mindestens 20 Volumen-% Sauerstoff enthält.

9. Gaszusammensetzung zur Verwendung
nach einem der Ansprüche 1 bis 8, **dadurch gekennzeichnet,**
**dass** die Neurointoxikation ausgewählt ist aus Excitotoxizitäten, welche einen Zustand der Abhängigkeit erzeugen, akuten Hirnschäden, neurodegenerativen Erkrankungen, und psychiatrischen oder neurologischen Pathologien.

10. Gaszusammensetzung zur Verwendung
nach einem der Ansprüche 1 bis 8, **dadurch gekennzeichnet,**
**dass** die Neurointoxikation resultiert aus oder Folge ist von einem Schädeltrauma, einem Schlaganfall (CVA), einer Hirnischämie, der Einnahme einer Abhängigkeit erzeugenden Substanz, der Alzheimer-Krankheit, der Parkinson-Krankheit, der Chorea Huntington, einer amyotrophen Lateralsklerose, einer akuten disseminierten Enzephalomyelitis, einer tardiven Dyskinesie, einer olivopontozerebellären Degeneration, einer Schizophrenie oder einer Epilepsie.

## Claims

1. Gaseous composition consisting of krypton in a proportion of 15 to 80% by volume and at least one additional gaseous compound chosen from oxygen, nitrogen and the mixtures thereof, for use as an inhalable drug intended to prevent or treat neurointoxication in a human being, wherein the gaseous krypton is the active principle.

2. Gaseous composition for use according to claim 1, **characterised in that** the neurointoxication is the result of a brain dysfunction of one or more neurotransmitter systems.

3. Gaseous composition for use according to one of claims 1 and 2, **characterised in that** said composition contains krypton in a proportion that is effective and sufficient for acting on at least one brain receptor in order to regulate the functioning of the dopamine, glutamate, serotonin, acetylcholine, taurine, GABA and/or noradrenaline neurotransmission systems.

4. Gaseous composition for use according to one of claims 1 to 3, **characterised in that** the proportion of krypton by volume is at least 30%.

5. Gaseous composition for use according to one of claims 1 to 4, **characterised in that** the proportion of krypton by volume is at most 75%.

6. Gaseous composition for use according to one of claims 1 to 5, **characterised in that** said composition contains air, preferably, the gaseous drug is ready to use.

7. Gaseous composition for use according to one of claims 1 to 6, **characterised in that** said composition consists of krypton and the rest oxygen, or krypton, nitrogen and oxygen.

8. Gaseous composition for use according to one of claims 1 to 7, **characterised in that** said composition contains at least 20% oxygen by volume.

9. Gaseous composition for use according to one of claims 1 to 8, **characterised in that** the neurointoxication is chosen from excitotoxicities that cause a state of addiction, acute cerebral accidents, neurodegenerative diseases, and psychiatric or neurological diseases.

10. Gaseous composition for use according to one of claims 1 to 8, **characterised in that** the neurointoxication is the result of or follows cranial trauma, a cerebrovascular accident (CVA), cerebral ischaemia, the taking of a substance that generates an addiction, Alzheimer's disease, Parkinson's disease, Huntington's chorea, amyotrophic lateral sclerosis, acute disseminated encephalomyelitis, tardive dyskinesia, olivopontocerebellar atrophy, schizophrenia or epilepsy.
